# EUROPEAN PATENT APPLICATION

(11) **EP 4 116 300 A1**
(43) Date of publication of application: **11.01.2023**
(21) Application number: 21764296.6
(22) Date of filing: 05.03.2021
(51) Int. Cl.: C07D 413/14, C07D 413/00, A61K 31/42, A61P 35/00

(54) **CRYSTAL OF TRICYCLIC COMPOUND ACTING ON CRBN PROTEIN AND PREPARATION METHOD THEREFOR**

(30) Priority: 06.03.2020 CN 202010153435
(71) Applicant: CHIA TAI TIANQING PHARMACEUTICAL GROUP CO., LTD., Lianyungang, Jiangsu 222062 (CN)
(72) Inventor: LEI, Maoyi, Shanghai 200131 (CN); XU, Yu, Shanghai 200131 (CN); LUO, Yunfu, Shanghai 200131 (CN); SHAN, Zhenliang, Lianyungang, Jiangsu 222062 (CN)
(74) Representative: Grünecker Patent- und Rechtsanwälte PartG mbB
(86) International application number: PCT/CN2021/079340
(87) International publication number: WO 2021/175317

(57) **Abstract**

Disclosed are a crystal of a tricyclic compound acting on CRBN protein and a preparation method therefor, specifically relating to a crystal of a compound of formula (I) and a preparation method therefor. An application of the crystal in the preparation of drugs for treating CRBN protein-related diseases is further comprised.

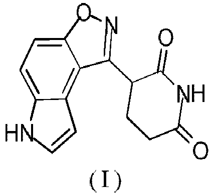

## Description

### CROSS-REFERENCE TO RELATED APPLICATIONS

The present application claims priority and benefit to the Chinese Patent Application No. 202010153435.5 filed with National Intellectual Property Administration, PRC on Mar. 06, 2020, the disclosure of which is incorporated herein by reference in its entirety.

### TECHNICAL FIELD

The present application belongs to the field of pharmaceutical chemistry, and relates to a crystalline form of a tricyclic compound acting on CRBN protein and a method for preparing the same, and particularly to a crystalline form of a compound of formula (I) and a method for preparing the same, as well as use of the crystalline form for preparing a medicament for treating a CRBN protein-associated disease.

### BACKGROUND

Thalidomide, sold under the trade name THALOMID^{®}, was first synthesized by Grünenthal Group of Germany. From the latter half of the 1950s to the early 1960s, it was sold as a sedative in over 40 countries and also widely used as an antiemetic for pregnant women, leading to the tragic birth of tens of thousands of infants with phocomelia (morphogenesis disorder) and ending up being withdrawn from the market.

After the thalidomide event, the mechanism of action of thalidomide teratogenicity has aroused great interest of researchers. Cereblon (CRBN) protein has been proved to be the target protein for thalidomide teratogenicity. Thalidomide forms an E3 ubiquitin ligase complex by combining with CRBN, Damaged DNA Binding Protein 1 (DDB1), Cullin-4A (CUL4A) and Regulator of Cullins 1 (ROC1) to ubiquitinate a plurality of substrate proteins and form ubiquitinated chains, so that the substrate proteins are recognized and hydrolyzed by proteasomes. Domide drugs, called Immunomodulatory Drugs (IMiDs), activate the E3 ubiquitin ligase complex formed with CRBN to ubiquitinate transcription factors IKZF1 and IKZF3, which are then recognized and degraded by proteasomes, thereby generating toxic effects on multiple myeloma. Absence of these two transcription factors will terminate the growth of myeloma. Domide drugs such as lenalidomide and pomalidomide are now first-line drugs for treating multiple myeloma.

CRBN is a protein that is conserved from plant to human and has 442 amino acids, and it is located on the short arm p26.3 of human chromosome 3, with a molecular weight of 51 kDa. In humans, CRBN gene has been identified as a candidate gene of Autosomal Recessive Inheritance of Non-Syndromic Mental Retardation (ARNSMR). CRBN is widely expressed in testis, spleen, prostate, liver, pancreas, placenta, kidney, lung, skeletal muscle, ovary, small intestine, peripheral blood leukocytes, colon, brain, and retina, and its expression in brain tissue (including retina) and testis is significantly higher than in other tissues.

Taking CRBN as an important target of anti-tumor and immune regulator drugs has been proved to have definite efficacy on various hematologic malignant tumors such as multiple myeloma and chronic lymphocytic leukemia, skin diseases such as erythema nodosum leprosum, and autoimmune diseases such as systemic lupus erythematosus. The domide drugs all have many side effects, especially peripheral neuropathy. There is an urgent need to develop CRBN modulator drugs with no teratogenic effect, fewer peripheral neuropathies, stronger immunomodulatory effect and higher anti-tumor activity to improve clinical therapeutic effects, reduce clinical side effects, and facilitate long-term use by patients.

### BRIEF SUMMARY

In one aspect, the present application provides a crystalline form of a compound of formula (I),

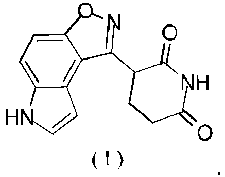

In another aspect, the present application provides a crystalline composition of the compound of formula (I), wherein the crystalline form of the compound of formula (I) described above makes up 50% or more, preferably 75% or more, more preferably 90% or more, and most preferably 95% or more, of the crystalline composition by weight.

In another aspect, the present application provides a pharmaceutical composition, which comprises a therapeutically effective amount of the crystalline form of the compound of formula (I) described above or the crystalline composition of the compound of formula (I) described above, and may comprise at least one pharmaceutically acceptable carrier or other excipient.

In another aspect, the present application provides use of the crystalline form of the compound of formula (I) described above, the crystalline composition of the compound of formula (I) described above, or the pharmaceutical composition described above for preparing a medicament for treating a CRBN protein-associated disease.

In another aspect, the present application provides use of the crystalline form of the compound of formula (I) described above, the crystalline composition of the compound of formula (I) described above, or the pharmaceutical composition described above for treating a CRBN protein-associated disease.

In another aspect, the present application provides a method for treating a CRBN protein-associated disease, which comprises administering to a mammal in need a therapeutically effective amount of the crystalline form of the compound of formula (I) described above, the crystalline composition of the compound of formula (I) described above, or the pharmaceutical composition described above.

In another aspect, the present application provides the crystalline form of the compound of formula (I) described above, the crystalline composition of the compound of formula (I) described above, or the pharmaceutical composition described above for use in treating a CRBN protein-associated disease.

### SUMMARY

In one aspect, the present application provides a crystalline form of a compound of formula (I),

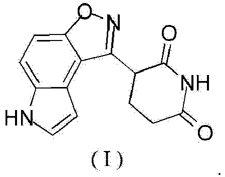

The crystalline form described herein may be present in the form of a non-solvate or a solvate, for example, a hydrate.

In one embodiment of the present application, the crystalline form of the compound of formula (I) is a crystalline form A, which has diffraction peaks at the following 2θ angles in an X-ray powder diffraction pattern using Cu-Ka radiation: 10.885±0.200°, 16.433±0.200° and 24.417±0.200°.

In one embodiment of the present application, the crystalline form A described above has diffraction peaks at the following 2θ angles in an X-ray powder diffraction pattern using Cu-Ka radiation: 10.885±0.200°, 16.433±0.200°, 18.052±0.200°, 18.942±0.200°, 21.124±0.200°, 21.740±0.200°, 24.417±0.200° and 29.034±0.200°.

In one embodiment of the present application, the crystalline form A described above has diffraction peaks at the following 2θ angles in an X-ray powder diffraction pattern using Cu-Ka radiation: 10.885±0.200°, 15.303±0.200°, 16.433±0.200°, 18.052±0.200°, 18.942±0.200°, 21.124±0.200°, 21.740±0.200°, 24.417±0.200°, 25.766±0.200°, 26.189±0.200°, 28.741±0.200° and 29.034±0.200°.

In one embodiment of the present application, the crystalline form A described above has diffraction peaks at the following 2θ angles in an X-ray powder diffraction pattern using Cu-Ka radiation: 10.885±0.200°, 15.303±0.200°, 16.433±0.200°, 18.052±0.200°, 18.942±0.200°, 21.124±0.200°, 21.740±0.200°, 24.417±0.200°, 25.766±0.200°, 26.189±0.200°, 28.741±0.200°, 29.034±0.200°, 31.241±0.200°, 32.588±0200°, 32.958±0.200°, 33.123±0.200°, 34.985±0.200°, 36.474±0.200° and 37.811±0.200°.

In some embodiments of the present application, the crystalline form A described above has diffraction peaks at the following 2θ angles in an X-ray powder diffraction pattern using Cu-Ka radiation: 10.885°±0.200°, 14.265°±0.200°, 15.303°±0.200°, 15.515°±0.200°, 16.433°±0.200°, 18.052°±0.200°, 18.942°±0.200°, 21.124°±0.200°, 21.740°±0.200°, 23.179°±0.200°, 23.781°±0.200°, 24.417°±0.200°, 25.766°±0.200°, 26.189°±0.200°, 28.741°±0.200°, 29.034°±0.200°, 31.241°±0.200°, 32.081°±0.200°, 32.588°±0.200°, 32.958°±0.200°, 33.123°±0.200°, 34.985°±0.200°, 36.474°±0.200°, 37.811°±0.200° and 39.622°±0.200°.

The present application provides a crystalline form A of a compound of formula (I), which comprises 3, 4, 5, 6, 7 or 8 diffraction peaks at 2θ angles selected from the group consisting of 10.885±0.200°, 16.433±0.200°, 18.052±0.200°, 18.942±0.200°, 21.124±0.200°, 21.740±0.200°, 24.417±0.200° and 29.034±0.200° in an X-ray powder diffraction pattern using Cu-Ka radiation.

The present application provides a crystalline form A of a compound of formula (I), which comprises 3, 4, 5, 6, 7, 8, 9, 10, 11 or 12 diffraction peaks at 2θ angles selected from the group consisting of 10.885±0.200°, 15.303±0.200°, 16.433±0.200°, 18.052±0.200°, 18.942±0.200°, 21.124±0.200°, 21.740±0.200°, 24.417±0.200°, 25.766±0.200°, 26.189±0.200°, 28.741±0.200° and 29.034±0.200° in an X-ray powder diffraction pattern using Cu-Ka radiation.

The present application provides a crystalline form A of a compound of formula (I), which comprises 3, 4, 5, 6, 7, 8, 9, 10, 11, 12 or more diffraction peaks at 2θ angles selected from the group consisting of 10.885±0.200°, 15.303±0.200°, 16.433±0.200°, 18.052±0.200°, 18.942±0.200°, 21.124±0.200°, 21.740±0.200°, 24.417±0.200°, 25.766±0.200°, 26.189±0.200°, 28.741±0.200°, 29.034±0.200°, 31.241±0.200°, 32.588±0.200°, 32.958±0.200°, 33.123±0.200°, 34.985±0.200°, 36.474±0.200° and 37.811±0.200° in an X-ray powder diffraction pattern using Cu-Ka radiation.

The present application provides a crystalline form A of a compound of formula (I), which comprises 3, 4, 5, 6, 7, 8, 9, 10, 11, 12 or more diffraction peaks at 2θ angles selected from the group consisting of 10.885°±0.200°, 14.265°±0.200°, 15.303°±0.200°, 15.515°±0.200°, 16.433°±0.200°, 18.052°±0.200°, 18.942°±0.200°, 21.124°±0.200°, 21.740°±0.200°, 23.179°±0.200°, 23.781°±0.200°, 24.417°±0.200°, 25.766°±0.200°, 26.189°±0.200°, 28.741°±0.200°, 29.034°±0.200°, 31.241°±0.200°, 32.081°±0.200°, 32.588°±0.200°, 32.958°±0.200°, 33.123°±0.200°, 34.985°±0.200°, 36.474°±0.200°, 37.811°±0.200° and 39.622°±0.200° in an X-ray powder diffraction pattern using Cu-Ka radiation.

In one embodiment of the present application, positions and intensities of the characteristic peaks in an X-ray powder diffraction pattern using Cu-Ka radiation of the crystalline form A of the compound of formula (I) are shown in Table 1:

**Table 1. XRPD pattern analysis data of crystalline form A of compound of formula (I)**

| **No.** | **2θ angle ±0.200 (°)** | **Relative intensity (%)** | **No.** | **2θ angle ±0.200 (°)** | **Relative intensity (%)** |
|---|---|---|---|---|---|
| 1 | 10.885 | 34.73 | 14 | 26.189 | 6.04 |
| 2 | 14.265 | 2.37 | 15 | 28.741 | 14.83 |
| 3 | 15.303 | 11.98 | 16 | 29.034 | 33.94 |
| 4 | 15.515 | 2.08 | 17 | 31.241 | 7.52 |
| 5 | 16.433 | 37.76 | 18 | 32.081 | 2.10 |
| 6 | 18.052 | 33.10 | 19 | 32.588 | 6.87 |
| 7 | 18.942 | 32.02 | 20 | 32.958 | 12.57 |
| 8 | 21.124 | 19.79 | 21 | 33.123 | 9.42 |
| 9 | 21.740 | 20.73 | 22 | 34.985 | 7.48 |
| 10 | 23.179 | 2.39 | 23 | 36.474 | 11.44 |
| 11 | 23.781 | 2.31 | 24 | 37.811 | 4.53 |
| 12 | 24.417 | 100.00 | 25 | 39.622 | 2.99 |
| 13 | 25.766 | 5.41 | 26 | / | / |

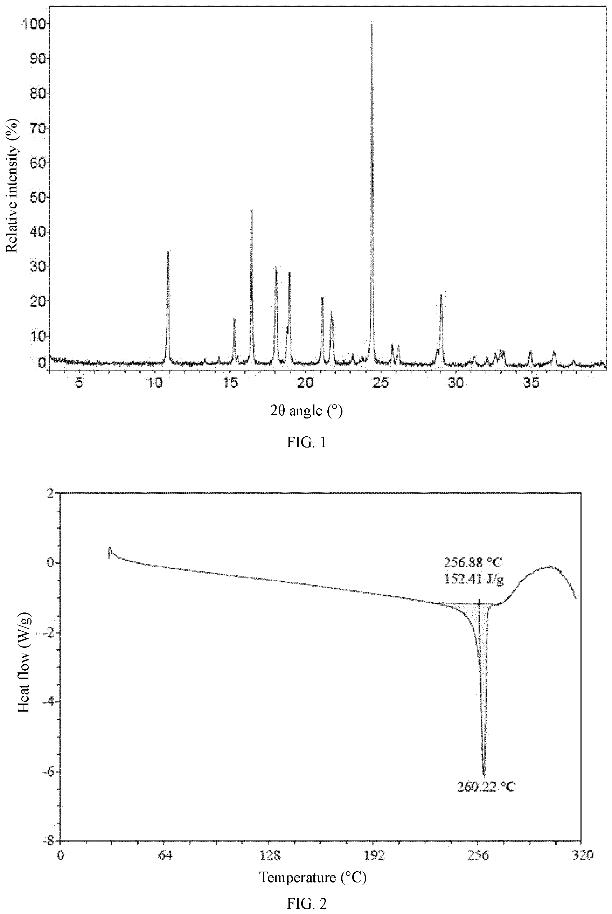

In one embodiment of the present application, the crystalline form A of the compound of formula (I) has an X-ray powder diffraction pattern using Cu-Ka radiation as shown in FIG. 1.

In one embodiment of the present application, the crystalline form A of the compound of formula (I) has a differential scanning calorimetry curve showing an endothermic peak at 260.2 °C.

In one embodiment of the present application, the endothermic peak in the differential scanning calorimetry curve of the crystalline form A of the compound of formula (I) starts at 256.9 °C.

In one embodiment of the present application, the crystalline form A of the compound of formula (I) has a differential scanning calorimetry (DSC) pattern as shown in FIG. 2.

In one embodiment of the present application, the crystalline form A of the compound of formula (I) has a thermogravimetric analysis curve showing a weight loss of 0.31% at 120.0 °C.

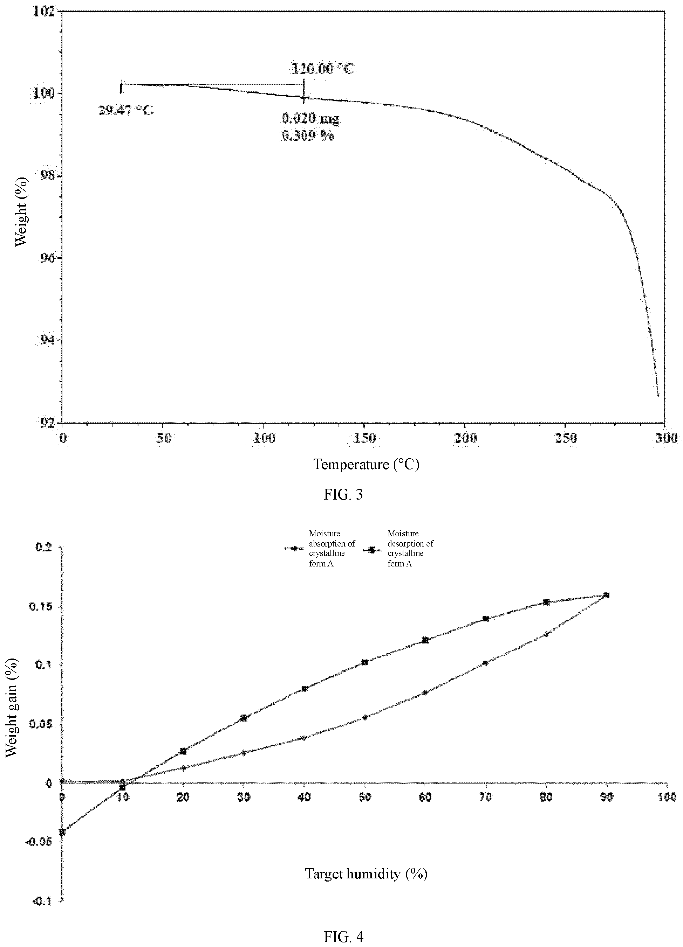

In one embodiment of the present application, the crystalline form A of the compound of formula (I) has a thermogravimetric analysis (TGA) pattern as shown in FIG. 3.

In one embodiment of the present application, the crystalline form A of the compound of formula (I) is prepared in an organic solvent.

In one embodiment of the present application, the crystalline form A of the compound of formula (I) is prepared by precipitation in an organic solvent.

In one embodiment of the present application, the crystalline form A of the compound of formula (I) has a hygroscopic weight gain of less than 0.2% at 25 °C with relative humidity at 80%.

In another aspect, the present application provides a crystalline form A of a compound of formula (I) prepared in an organic solvent, wherein the crystalline form A of the compound of formula (I) has the crystalline characteristics of the crystalline form A described above.

In one embodiment of the present application, the present application provides a crystalline form A of a compound of formula (I) prepared by precipitation in an organic solvent, wherein the crystalline form A of the compound of formula (I) has the crystalline characteristics of the crystalline form A described above.

In another aspect, the present application provides a method for preparing the crystalline form A of the compound of formula (I), which comprises a step of precipitating the compound of formula (I) from an organic solvent.

In one embodiment of the present application, the organic solvent described above is selected from the group consisting of one or more of tetrahydrofuran, dimethyl sulfoxide, acetonitrile, *n*-heptane, methyl *tert-butyl* ether, ethyl acetate and isopropanol.

In one embodiment of the present application, the organic solvent is selected from a solvent mixture of tetrahydrofuran and methyl *tert*-butyl ether.

In one embodiment of the present application, the crystalline form A of the compound of formula (I) may be prepared under stirring.

In another aspect, the present application provides a method for preparing the crystalline form A of the compound of formula (I), which comprises a step of precipitating the compound of formula (I) from a solvent mixture of a good solvent and a poor solvent.

In one embodiment of the present application, the present application provides a method for preparing the crystalline form A of the compound of formula (I), which comprises:
(a) adding the compound of the formula (I) into a good solvent to form a solution of the compound of the formula (I);
(b) slowly adding dropwise a poor solvent under stirring at room temperature; and
(c) precipitating a solid.

In one embodiment of the present application, the good solvent in step (a) described above is selected from the group consisting of tetrahydrofuran and dimethyl sulfoxide.

In one embodiment of the present application, the poor solvent in step (b) described above is selected from the group consisting of acetonitrile, *n*-heptane, methyl *tert-butyl* ether, ethyl acetate and isopropanol.

In one embodiment of the present application, the good solvent in step (a) described above is selected from tetrahydrofuran, and the poor solvent in step (b) described above is selected from methyl *tert*-butyl ether.

In one embodiment of the present application, the mass-to-volume ratio of the compound of formula (I) to the good solvent in step (a) described above is (25-100) mg:1 mL.

In one specific embodiment of the present application, the mass-to-volume ratio of the compound of formula (I) to the good solvent in step (a) described above is 25 mg:1 mL.

In one embodiment of the present application, the volume ratio of the good solvent in step (a) to the poor solvent in step (b) described above is 1:(1-15).

In one specific embodiment of the present application, the volume ratio of the good solvent in step (a) to the poor solvent in step (b) described above is 1:10.

In one embodiment of the present application, step (c) described above further comprises filtering, centrifuging and drying the precipitated solid.

In another aspect, the present application further provides a method for preparing the crystalline form A of the compound of formula (I), which comprises:
(1) slurrying the compound of formula (I) in an organic solvent; and
(2) cooling the slurry to room temperature and performing filtration and concentration to give the crystalline form A of the compound of the formula (I).

In one embodiment of the present application, the solvent in step (1) described above is selected from the group consisting of acetonitrile, *n*-heptane, methyl *tert-butyl* ether, ethyl acetate and isopropanol.

In another aspect, the present application further provides a method for preparing the crystalline form A of the compound of formula (I), which comprises:
(i) adding the compound of formula (I) to an organic solvent and heating the resulting mixture until the compound of formula (I) is completely dissolved; and
(ii) cooling and stirring the solution and performing filtration to give the crystalline form A of the compound of the formula (I).

In one embodiment of the present application, the organic solvent in step (i) described above is selected from *N*,*N*-dimethylformamide (DMF).

In one embodiment of the present application, the mass-to-volume ratio of the compound of formula (I) to the organic solvent in step (i) described above is (0.1-1) g:1 mL, preferably (0.2-0.5) g:1 mL, and more preferably (0.2-0.33) g:1 mL.

In one embodiment of the present application, step (ii) described above further comprises drying the solid obtained by filtration.

In another aspect, the present application provides a crystalline composition of the compound of formula (I), wherein the crystalline form of the compound of formula (I) described above makes up 50% or more, preferably 75% or more, more preferably 90% or more, and most preferably 95% or more, of the crystalline composition by weight. The crystalline composition may also comprise other crystalline or amorphous forms of the compound of formula (I) in small amounts.

In another aspect, the present application provides a pharmaceutical composition, which comprises a therapeutically effective amount of the crystalline form of the compound of formula (I) described above or the crystalline composition of the compound of formula (I) described above, and may comprise at least one pharmaceutically acceptable carrier or other excipient.

In one embodiment of the present application, the pharmaceutical composition is selected from the group consisting of pharmaceutical compositions for oral, subcutaneous, intramuscular and intravenous administration. In one embodiment of the present application, the pharmaceutical composition is selected from the group consisting of pharmaceutical compositions for oral, intramuscular and intravenous administration.

In one embodiment of the present application, the pharmaceutical composition is selected from pharmaceutical compositions for oral administration.

In another aspect, the present application provides an oral pharmaceutical composition, which comprises a therapeutically effective amount of the crystalline form of the compound of formula (I) described above or the crystalline composition of the compound of formula (I) described above, and may comprise at least one pharmaceutically acceptable carrier or other excipient.

In one embodiment of the present application, the pharmaceutical composition is selected from the group consisting of tablets, capsules, granules, pulvises, pills, powders, pastilles, syrups and suspensions.

In one embodiment of the present application, the pharmaceutical composition is selected from the group consisting of tablets, capsules and granules.

In one embodiment of the present application, the pharmaceutical composition is selected from the group consisting of aqueous and non-aqueous solutions for injection.

In one embodiment of the present application, the pharmaceutical composition is selected from the group consisting of a rapid-release, delayed-release and modified-release pharmaceutical compositions.

In one embodiment of the present application, the pharmaceutical composition is selected from the group consisting of rapid-release pharmaceutical compositions.

In one embodiment of the present application, the pharmaceutical composition is selected from the group consisting of rapid-release oral pharmaceutical compositions.

In another aspect, the present application provides use of the crystalline form of the compound of formula (I) described above, the crystalline composition of the compound of formula (I) described above, or the pharmaceutical composition described above for preparing a medicament for treating a CRBN protein-associated disease.

In another aspect, the present application provides use of the crystalline form of the compound of formula (I) described above, the crystalline composition of the compound of formula (I) described above, or the pharmaceutical composition described above for treating a CRBN protein-associated disease.

In another aspect, the present application provides a method for treating a CRBN protein-associated disease, which comprises administering to a mammal in need a therapeutically effective amount of the crystalline form of the compound of formula (I) described above, the crystalline composition of the compound of formula (I) described above, or the pharmaceutical composition described above.

In another aspect, the present application provides the crystalline form of the compound of formula (I) described above, the crystalline composition of the compound of formula (I) described above, or the pharmaceutical composition described above for use in treating a CRBN protein-associated disease.

In one embodiment of the present application, the mammal is a human.

In one embodiment of the present application, the CRBN protein-associated disease is selected from multiple myeloma.

In another aspect, the present application also provides a crystalline form of a compound of formula (I) that has a hygroscopic weight gain ΔW% of less than 0.2% at 25 °C with RH at 80%, wherein the crystalline form is the crystalline form A of the compound of formula (I) described above.

The crystalline form of the compound of the formula (I) described herein has stable properties, low hygroscopicity, and good druggability. The crystalline form of the compound of the formula (I) described herein also has an excellent inhibitory effect on cell proliferation, a significant inhibitory effect and a reducing effect on tumors, and high oral bioavailability.

In the present application, the pharmaceutical composition can be formulated into a certain dosage form, and the administration route is preferably oral administration, parenteral administration (including subcutaneous, intramuscular and intravenous administration), rectal administration, and the like. For example, suitable dosage forms for oral administration include tablets, capsules, granules, pulvises, pills, powders, pastilles, syrups or suspensions; suitable dosage forms for parenteral administration include aqueous or non-aqueous solutions or emulsions for injection; suitable dosage forms for rectal administration include suppositories with hydrophilic or hydrophobic carriers. The dosage forms may also be formulated as desired for rapid, delayed or modified release of the active ingredient.

In the present application, the X-ray powder diffraction pattern of a sample is determined in the following conditions: instrument: Bruker D8 ADVANCE X-ray diffractometer; target: Cu:Kα; wavelength λ = 1.54060 Å; range of 2θ: 3-40°; scattering slit: 0.60 mm; detector slit: 10.50 mm; anti-scatter slit: 7.10 mm; scanning rate: 10 deg/min; sample rotation speed: 15 rpm; Cu-target tube voltage and current: 40 kV, 40 mA.

In the present application, the DSC spectrum is determined under the following conditions: instrument: TA Q2000 differential scanning calorimeter; temperature range: 30-320 °C; heating rate: 10 °C/min.

In the present application, the thermogravimetric analysis (TGA) is performed under the following conditions: instrument: DISCOVERY TGA 5500 thermogravimetric analyzer; temperature range: 25-300 °C; heating rate: 10 °C/min.

In the present application, dynamic vapor sorption (DVS) analysis is performed under the following conditions: instrument: SMS DVS Advantage-1; equilibration: dm/dt = 0.01%/min (shortest: 60 min, longest: 180 min); drying: drying at 0% RH for 120 min; RH (%) test gradient: 10%; range of RH (%) test gradient: 0%-90%-0%.

It should be noted that in the X-ray powder diffraction pattern, the position and relative intensity of a peak may vary due to measuring instruments, measuring methods/conditions, and other factors. For any specific crystalline form, the position of a peak may have an error, and the measurement of 2θ has an error of ±0.2°. Therefore, this error should be considered when determining each crystalline form, and crystalline forms within this margin of error are within the scope of the present application.

It should be noted that, for the same crystalline form, the position of an endothermic peak in the DSC (differential scanning calorimetry) pattern may vary due to measuring instruments, measuring methods/conditions, and other factors. For any specific crystalline form, the position of an endothermic peak may have an error of ±5 °C, ±3 °C, or ±2 °C. Therefore, this error should be considered when determining each crystalline form, and crystalline forms within this margin of error are within the scope of the present application.

It should be noted that, for the same crystalline form, the position of a weight loss temperature in the TGA pattern may vary due to measuring instruments, measuring methods/conditions, and other factors. For any specific crystalline form, the weight loss temperature may have an error of ±5 °C, ±3 °C, or ±2 °C. Therefore, this error should be considered when determining each crystalline form, and crystalline forms within this margin of error are within the scope of the present application.

### Definitions and Description

Unless otherwise stated, the following terms and phrases used herein are intended to have the following meanings. A particular phrase or term, unless otherwise specifically defined, should not be considered as uncertain or unclear, but construed according to its common meaning. When referring to a trade name, it is intended to refer to its corresponding commercial product or its active ingredient.

"Mammal" includes human, domestic animals such as laboratory mammals and domestic pets (e.g., cat, dog, pig, cow, sheep, goat, horse, rabbit), and non-domesticated mammals such as wild mammals.

The term "pharmaceutical composition" refers to a formulation of the compound disclosed herein with a vehicle commonly recognized in the art for delivering a biologically active compound to a mammal, such as a human. The vehicle includes all pharmaceutically acceptable carriers for its use. The pharmaceutical composition facilitates administration of the compound to an organism.

The term "therapeutically effective amount" refers to an amount of a drug or a medicament that is sufficient to provide the desired effect but is non-toxic. The determination of the effective amount varies from person to person. It depends on the age and general condition of a subject, as well as the particular active substance used. The appropriate effective amount in a case may be determined by those skilled in the art in the light of conventional tests.

The term "treating" or "treatment" means administering the compound or formulation described herein to ameliorate or eliminate a disease or one or more symptoms associated with the disease, and includes:
(i) inhibiting a disease or disease state, i.e., arresting its development; and
(ii) alleviating a disease or disease state, i.e., causing its regression.

The term "pharmaceutically acceptable carriers" refers to those which are administered together with the active ingredient, do not have a significant irritating effect on an organism and do not impair the biological activity and properties of the active compound. For additional information on carriers, reference may be made to Remington:

The Science and Practice of Pharmacy, 21st Ed., Lippincott, Williams & Wilkins (2005), the content of which is incorporated herein by reference.

In the present application, "room temperature" refers to 20 °C-30 °C.

The word "comprise" and variations thereof such as "comprises" or "comprising" will be understood in an open, non-exclusive sense, i.e., "including but not limited to".

### BRIEF DESCRIPTION OF THE DRAWINGS

FIG. 1 is an X-ray powder diffraction (XRPD) pattern of the crystalline form A of the compound of formula (I).
FIG. 2 is a differential scanning calorimetry (DSC) pattern of the crystalline form A of the compound of formula (I).
FIG. 3 is a thermogravimetric analysis (TGA) pattern of the crystalline form A of the compound of formula (I).
FIG. 4 is a dynamic vapor sorption (DVS) pattern of the crystalline form A of the compound of formula (I).
FIG. 5 shows changes in the IKZF3 protein level in cells as determined by WB after multiple myeloma cells MM.1S are treated with the compound of formula (I) at concentrations of 50 and 500 nM.

### DETAILED DESCRIPTION

In order to better understand the content of the present application, further description is given with reference to specific examples, but the specific embodiments are not intended to limit the content of the present application.

### Example 1: Preparation of the Compound of Formula (I)

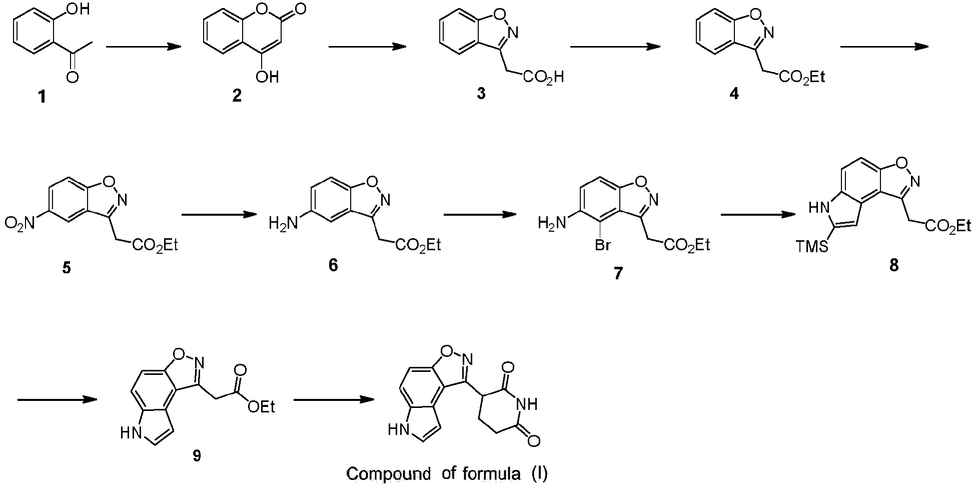

### Step 1: Synthesis of intermediate 2

Compound **1** (25.00 g, 183.62 mmol) was dissolved in a mixture of diethyl carbonate (250 mL) and toluene (250 mL) at room temperature. The reaction mixture was cooled to 0 °C, and sodium hydride (36.72 g, 918.12 mmol, 60% purity) was added in batches. The reaction mixture was heated to 120 °C and stirred for 12 h. After the reaction was completed, the reaction mixture was cooled to room temperature and poured slowly into ice water (500 mL), and ethyl acetate (500 mL × 2) was added for extraction. The organic phase was discarded, and the aqueous phase was adjusted to pH 3 with 3 N hydrochloric acid and extracted with ethyl acetate (1 L × 3). The organic phases were combined, dried over anhydrous sodium sulfate and filtered, and the filtrate was concentrated under reduced pressure to remove the solvent and thus to give intermediate 2. ¹H NMR (400 MHz, DMSO_*d₆*) δ: 12.52 (s, 1H), 7.82 (dd, *J* = 1.6, 7.6 Hz, 1H), 7.68-7.62 (m, 1H), 7.40-7.32 (m, 2H), 5.59 (s, 1H).

### Step 2: Synthesis of intermediate 3

Intermediate **2** (25.00 g, 154.19 mmol) was dissolved in methanol (300 mL) at room temperature, and sodium acetate (44.27 g, 539.65 mmol) and hydroxylamine hydrochloride (37.50 g, 539.65 mmol) were added successively. The reaction mixture was stirred at 15 °C for 1.5 h and then stirred at 80 °C for 12 h. After the reaction was completed, the reaction mixture was cooled to room temperature, adjusted to pH = 5 with 3 N hydrochloric acid, and then concentrated under reduced pressure to remove the solvent. Water (200 mL) was added, and the reaction flask was placed in an ice-water bath to cool while 3 N hydrochloric acid was added to adjust the pH to 3. After being stirred for 30 min, the reaction mixture was filtered, and the filter cake was collected and concentrated under reduced pressure to remove the solvent and thus to give intermediate **3.**

### Step 3: Synthesis of intermediate 4

Intermediate **3** (28.00 g, 158.05 mmol) was dissolved in ethanol (600 mL) at room temperature, and concentrated sulfuric acid (28 mL, 98% purity) was added. The reaction mixture was heated to 90 °C and stirred for 6 h. After the reaction was completed, the reaction mixture was cooled to room temperature and concentrated under reduced pressure to remove the solvent. The resulting residue was diluted with ethyl acetate (300 mL) and water (300 mL), and solid sodium bicarbonate was added to adjust the pH to 7. After liquid separation, the organic phase was collected and the aqueous phase was extracted with ethyl acetate (300 mL × 2). The organic phases were combined, washed with saturated brine (300 mL), dried over anhydrous sodium sulfate, and filtered, and the filtrate was concentrated under reduced pressure to remove the solvent. The resulting residue was purified by column chromatography (eluent: petroleum ether/ethyl acetate = 1/0 to 0/1, volume ratio) to give intermediate **4.** ¹H NMR (400 MHz, DMSO_*d₆*) δ: 7.84 (d, *J* = 8.0 Hz, 1H), 7.74 (d, *J* = 8.4 Hz, 1H), 7.70-7.63 (m, 1H), 7.44-7.37 (m, 1H), 4.21 (s, 2H), 4.14 (q, *J* = 7.2 Hz, 2H), 1.19 (t, *J* = 7.2 Hz, 3H).

### Step 4: Synthesis of intermediate 5

Intermediate **4** (24.00 g, 116.95 mmol) was added to fuming nitric acid (180 mL) at 0 °C. The reaction mixture was stirred at 0 °C for 2 h. After the reaction was completed, the reaction mixture was poured into ice water (500 mL), and ethyl acetate (500 mL × 3) was added for extraction. The organic phases were combined, washed with water (200 mL × 2), dried over anhydrous sodium sulfate and filtered, and the filtrate was concentrated under reduced pressure to remove the solvent. The resulting residue was purified by column chromatography (eluent: petroleum ether/ethyl acetate = 1/0 to 10/1, volume ratio) to give intermediate **5.** ¹H NMR (400 MHz, DMSO_*d₆*) δ: 8.93 (d, *J* = 2.4 Hz, 1H), 8.51 (dd, *J* = 2.0, 9.2 Hz, 1H), 7.99 (d, *J* = 9.2 Hz, 1H), 4.34 (s, 2H), 4.15 (q, *J* = 7.4 Hz, 2H), 1.2 (t*, J* = 7.0 Hz, 3H).

### Step 5: Synthesis of intermediate 6

Intermediate 5 (5.50 g, 21.98 mmol) was dissolved in ethanol (120 mL) at room temperature, and stannic chloride dihydrate (19.84 g, 87.93 mmol) was added. The reaction mixture was stirred at 15 °C for 2 h. The reaction mixture was supplemented with stannic chloride dihydrate (14.88 g, 65.95 mmol) and then stirred for 5 h. After the reaction was completed, the reaction mixture was concentrated under reduced pressure to remove the solvent. The resulting residue was diluted with dichloromethane (200 mL) and water (300 mL), and then solid sodium bicarbonate was added to adjust the pH to 7. The resulting solution was filtered through a funnel containing diatomite. The diatomite was rinsed with 2-methyltetrahydrofuran (1000 mL), and the filtrate was collected. After liquid separation, the organic phase was collected, and the aqueous phase was extracted with 2-methyltetrahydrofuran (200 mL × 3). The organic phases were combined, dried over anhydrous sodium sulfate and filtered, and the filtrate was concentrated under reduced pressure to remove the solvent. The resulting residue was purified by column chromatography (a neutral alumina column, 200-300 mesh, eluent: petroleum ether/ethyl acetate/dichloromethane = 1/0/0 to 6/3/1, volume ratio) to give intermediate **6.**

### Step 6: Synthesis of intermediate 7

Intermediate **6** (1.50 g, 6.81 mmol) was dissolved in dichloromethane (30 mL) at room temperature, and *N*-bromosuccinimide (1.21 g, 6.81 mmol) was added at 0 °C. The reaction mixture was stirred at 0-15 °C for 2 h. After the reaction was completed, water (15 mL) was added to the reaction mixture, and dichloromethane (20 mL × 3) was then added for extraction. The organic phases were combined, washed with saturated brine (20 mL), dried over anhydrous sodium sulfate, and filtered, and the filtrate was concentrated under reduced pressure to remove the solvent and thus to give intermediate 7. ¹H NMR (400 MHz, DMSO_*d₆*) δ: 7.51 (d, *J* = 8.8 Hz, 1H), 7.16 (d, *J* = 8.8 Hz, 1H), 5.42 (s, 2H), 4.20-4.08 (m, 4H), 1.20 (t*, J* = 7.0 Hz, 3H).

### Step 7: Synthesis of intermediate 8

Intermediate **7** (1.1 g, 3.68 mmol) was dissolved in *N*,*N-*dimethylformamide (10 mL) at room temperature under nitrogen atmosphere, and trimethylsilylacetylene (722.38 mg, 7.36 mmol), triethylamine (930.30 mg, 9.20 mmol), potassium iodide (610.46 mg, 3.68 mmol), copper(I) iodide (70.04 mg, 368.00 µmol) and [1,1'-bis(triphenylphosphino)ferrocene]dichloropalladium(II) dichloromethane (300.31 mg, 368.00 µmol) were added. The reaction mixture was heated to 110 °C and reacted under microwave (3 bar) for 3 h. After the reaction was completed, the reaction mixture was cooled to room temperature. Water (30 mL) was added, and ethyl acetate (30 mL × 3) was added for extraction. The organic phases were combined, washed with half-saturated brine (20 mL), dried over anhydrous sodium sulfate and filtered, and the filtrate was concentrated under reduced pressure to remove the solvent. The resulting residue was purified by column chromatography (eluent: petroleum ether/ethyl acetate = 3/1, volume ratio). The resulting residue was purified by prep-HPLC (mobile phase: acetonitrile/water; acidic system: 0.05% HCl) to give intermediate **8.** ¹H NMR (400 MHz, DMSO_*d₆*) δ: 11.63 (s, 1H), 7.69 (d, *J* = 9.2 Hz, 1H), 7.42 (d, *J* = 9.2 Hz, 1H), 6.79 (d, *J* = 1.2 Hz, 1H), 4.26 (s, 2H), 4.15 (q, *J* = 7.0 Hz, 2H), 1.20 (t, *J* = 7.0 Hz, 3H), 0.34 (s, 9H).

### Step 8: Synthesis of intermediate 9

Intermediate **8** (300.00 mg, 948.09 µmol) was dissolved in acetonitrile (20 mL) at room temperature, and a solution of tetrabutylammonium fluoride (1 M, 948.09 µL) in tetrahydrofuran was added. The reaction mixture was heated to 70 °C and stirred for 2 h. After the reaction was completed, the reaction mixture was cooled to room temperature and concentrated under reduced pressure to remove the solvent. The resulting residue was purified by column chromatography (eluent: petroleum ether/ethyl acetate = 1/0 to 2/1, volume ratio) to give intermediate **9.** ¹H NMR (400 MHz, DMSO_*d₆*) δ: 11.69 (s, 1H), 7.71 (d, *J* = 8.8 Hz, 1H), 7.55 (t*, J* = 2.8 Hz, 1H), 7.42 (d, *J* = 8.8 Hz, 1H), 6.63 (s, 1H), 4.26 (s, 2H), 4.14 (q, *J* = 7.2 Hz, 2H), 1.17 (t*, J* = 7.0 Hz, 3H).

### Step 9: Synthesis of compound of formula (I)

Intermediate **9** (125.00 mg, 511.78 µmol) was dissolved in tetrahydrofuran (7 mL) at 0 °C, and acrylamide (36.38 mg, 511.78 µmol) and potassium *tert*-butoxide (57.43 mg, 511.78 µmol) were added successively. The reaction mixture was warmed to room temperature of 10 °C and stirred for 1 h. After the reaction was completed, water (10 mL) was added to the reaction mixture, and 2-methyltetrahydrofuran (20 mL × 3) was added for extraction. The organic phases were combined, washed with saturated brine (10 mL), dried over anhydrous sodium sulfate, and filtered, and the filtrate was concentrated under reduced pressure to remove the solvent. The resulting residue was purified by prep-HPLC (mobile phase: acetonitrile/water; acidic system: 0.05% HCl) to give the target compound, the compound of formula (I). MS-ESI *m*/*z:* 270.1 [M+H]⁺ ; ¹H NMR (400 MHz, DMSO_*d₆*) δ: 11.71 (s, 1H), 11.20 (s, 1H), 7.72 (d, *J* = 8.8 Hz, 1H), 7.56 (t, *J* = 2.6 Hz, 1H), 7.44 (d, *J* = 9.2 Hz, 1H), 6.56 (s, 1H), 4.69 (dd, *J* = 5.0, 11.8 Hz, 1H), 2.90-2.81 (m, 1H), 2.70-2.59 (m, 1H), 2.47-2.37 (m, 1H), 2.28-2.15 (m, 1H).

### Example 2: Preparation of Crystalline Form A of the Compound of Formula (I)

50 mg of the compound of formula (I) was weighed into a glass flask. 2 mL of tetrahydrofuran was added at room temperature to form a solution of the compound of formula (I) in tetrahydrofuran. 20 mL of methyl *tert-butyl* ether was slowly added dropwise at room temperature with stirring, and a solid precipitated. The mixture was centrifuged and filtered. The resulting solid was dried in a vacuum oven at 30 °C for 2 h to give the crystalline form A of the compound of formula (I).

### Example 3: Preparation of Crystalline Form A of the Compound of Formula (I)

6 mL of DMF was added to 2 g of the compound of the formula (I). The mixture was heated to 85-90 °C, then naturally cooled to room temperature, stirred for 36 h, and filtered. The filter cake was rinsed with 6 mL of *n*-heptane and dried *in vacuo* at 40-45 °C for 5 h to give the crystalline form A of the compound of formula (I).

### Experimental Example 1: Hygroscopicity Study of Crystalline Form A of the Compound of Formula (I)

The evaluation of hygroscopicity was classified as follows:

| **Classification of hygroscopicity** | ΔW% |
|---|---|
| Deliquescence | Absorb sufficient water to form a solution |
| Very hygroscopic | ΔW% ≥ 15% |
| Hygroscopic | 15%>ΔW%≥2% |
| Slightly hygroscopic | 2% > ΔW% ≥ 0.2% |
| Non-hygroscopic or hardly hygroscopic | ΔW% < 0.2% |

| | |
|---|---|
| Note: ΔW% represents the hygroscopic weight gain of the test compound at 25±1 °C and 80±2% RH. | |

### Experimental instruments:

Dynamic Vapor Sorption Apparatus SMS DVS Advantage-1

### Method:

10-15 mg of the crystalline form A of the compound of formula (I) was placed in a DVS sample tray and tested.

### Experimental results:

The DVS pattern of the crystalline form A of the compound of formula (I) is shown in FIG. 4, ΔW = 0.13%.

### Conclusion:

The crystalline form A of the compound of formula (I) had a hygroscopic weight gain of 0.13% at 25 °C and 80% RH, which is less than 0.2%, indicating no or almost no hygroscopicity. The crystalline form remained unchanged.

### Experimental Example 2: Stability Test of Crystalline Form A of the Compound of Formula (I) in Different Solvents

50-mg portions of the crystalline form A of the compound of formula (I) were weighed into different 2.0 mL glass vials, and appropriate amounts of single or mixed solvents in the table below were added. After the addition of magnetic stir bars, the samples were stirred on a magnetic stirrer (25 °C/50 °C, 700 rpm) in the dark. The 25 °C samples were stirred for 1 week and centrifuged, and the resulting solid samples were dried in a vacuum oven at 40 °C overnight. The 50 °C samples were stirred for 2 days and centrifuged in a centrifuge tube with a filter membrane, and the solvent in the lower layer was discarded. The samples were let stand in a fume hood for 1 h so that they were air-dried. The resulting dried samples were subjected to XRPD analysis to determine their crystalline forms. The results are shown in Table **2.**

**Table 2. Stability test of crystalline form A of the compound of formula (I) in different solvents**

| No. | Solvent (volume ratio) | Test results | | XRPD results | |
|---|---|---|---|---|---|
| | | 25 °C (after 1 week) | 50 °C (after 2 days) | 25 °C | 50 °C |
| 1 | Methanol | White suspension | White suspension | Crystalline form A | Crystalline form A |
| 2 | Ethanol | White suspension | White suspension | Crystalline form A | Crystalline form A |
| 3 | Acetone | White suspension | White suspension | Crystalline form A | Crystalline form A |
| 4 | Acetonitrile | White suspension | White suspension | Crystalline form A | Crystalline form A |
| 5 | Tetrahydrofuran | White suspension | White suspension | Crystalline form A | Crystalline form A |
| 6 | Methyl *tert*-butyl ether | White suspension | White suspension | Crystalline form A | Crystalline form A |
| 7 | Ethyl acetate | White suspension | White suspension | Crystalline form A | Crystalline form A |
| 8 | Water | White suspension | White suspension | Crystalline form A | Crystalline form A |
| 9 | Acetonitrile:water (1:1, volume ratio) | White suspension | White suspension | Crystalline form A | Crystalline form A |
| 10 | Acetone:water (1:1, volume ratio) | White suspension | White suspension | Crystalline form A | Crystalline form A |

| | | | | | |
|---|---|---|---|---|---|
| Conclusion: The crystalline form A of the compound of formula (I) has good stability in such solvents as methanol, ethanol, acetone, acetonitrile, tetrahydrofuran, methyl *tert-butyl* ether, ethyl acetate, water, and a solvent mixture of acetonitrile or acetone and water. | | | | | |

### Experimental Example 3: Solid Stability Test of Crystalline Form A of the Compound of Formula (I) at High Temperature, at High Humidity, and in Intense Light

According to the "Guidelines for the Stability Test of APIs and Preparations" (General Principles 9001 of the Four Parts of the Chinese Pharmacopoeia, 2020 Edition), the stability of the crystalline form A of the compound of formula (I) was investigated at high temperature (60 °C, open), at high humidity (room temperature/relative humidity at 92.5%, open), and in intense light (5000±500 Lux, 90 µw/cm², open).

1.5 g of the crystalline form A of the compound of formula (I) was weighed into an open measuring flask and spread out to form a thin layer. The sample for high temperature test was placed in an electrothermal drying oven and investigated, and the sample for high humidity test was placed in an integrated drug stability test chamber and investigated. Samples were taken on days 5, 10 and 30 for analysis, and the results were compared with the preliminary results of day 0. The sample for intense light test was placed in an open flask. Samples were taken on days 5 and 10 for analysis, and the results were compared with the preliminary results of day 0. The experimental results are shown in Table **3** below.

**Table 3. Solid stability test results of crystalline form A of the compound of formula (I) at high temperature, at high humidity, and in intense light**

| Conditions | Time point of sampling | Appearance | Content | Total impurities |
|---|---|---|---|---|
| - | Day 0 | Light green powder | 98.9% | <0.05% |
| High temperature (60 °C, open) | Day 5 | Light green powder | 99.9% | <0.05% |
| | Day 10 | Light green powder | 100.2% | <0.05% |
| | Day 30 | Light green powder | 100.2% | <0.05% |
| High humidity (room temperature/relative humidity 92.5%, open) | Day 5 | Light green powder | 100.7% | <0.05% |
| | Day 10 | Light green powder | 100.2% | <0.05% |
| | Day 30 | Light green powder | 100.4% | <0.05% |
| Intense light (5000±500 Lux, 90 µw/cm², open) | Day 5 | Yellowish-green powder | 100.7% | <0.05% |
| | Day 10 | Yellowish-green powder | 100.0% | <0.05% |

| | | | | |
|---|---|---|---|---|
| Conclusion: The crystalline form A of the compound of formula (I) has good stability at high temperature, at high humidity, and in intense light. | | | | |

### Experimental Example 4: Solid Stability Test of Crystalline Form A of the Compound of Formula (I) Under Accelerated Conditions

According to the "Guidelines for the Stability Test of APIs and Preparations" (General Principles 9001 of the Four Parts of the Chinese Pharmacopoeia, 2020 Edition), the stability of the crystalline form A of the compound of formula (I) was investigated under accelerated conditions at high temperature and high humidity (40 °C/relative humidity at 75%, sealed).

1.5 g of the crystalline form A of the compound of formula (I) was weighed into a double-layer low-density polyethylene bag. Each layer of the low-density polyethylene bag was sealed with a binding tie, and the bag was put into an aluminum foil bag. Samples were taken in months 1, 2, 3 and 6 for analysis, and the results were compared with the preliminary results of day 0. This experiment was repeated twice, each with a different batch of the crystalline form A of the compound of formula (I). The experimental results are shown in Table 4 below.

**Table 4. Solid stability test results of crystalline form A of the compound of formula (I) under accelerated conditions (40 °C/relative humidity at 75%, sealed)**

| Conditions | Batch | Time point of sampling | Appearance | Content | Total impurities | Crystalline form (XRPD) |
|---|---|---|---|---|---|---|
| - | 1 | Day 0 | Light green powder | 98.9% | <0.05% | Crystalline form A |
| 40 °C/relative humidity at 75%, sealed | | Month 1 | Light green powder | 100.1% | 0.05% | Not detected |
| | | Month 2 | Light green powder | 100.4% | <0.05% | Not detected |
| | | Month 3 | Light green powder | 100.4% | <0.05% | Crystalline form A |
| | | Month 6 | Light green powder | 98.1% | <0.05% | Crystalline form A |
| - | 2 | Day 0 | White powder | 100.2% | <0.05% | Crystalline form A |
| 40 °C/relative humidity at 75%, sealed | | Month 1 | Light green powder | 100.0% | 0.05% | Not detected |
| | | Month 2 | Light green powder | 100.2% | <0.05% | Not detected |
| | | Month 3 | Light green powder | 100.2% | <0.05% | Crystalline form A |
| | | Month 6 | Light green powder | 99.2% | <0.05% | Crystalline form A |

| | | | | | | |
|---|---|---|---|---|---|---|
| Conclusion: The crystalline form A of the compound of formula (I) has good stability under accelerated conditions at 40 °C with relative humidity at 75%. | | | | | | |

### Experimental Example 5: Solid Stability Test of Crystalline Form A of the Compound of Formula (I) Under Long-Term Conditions

According to the "Guidelines for the Stability Test of APIs and Preparations" (General Principles 9001 of the Four Parts of the Chinese Pharmacopoeia, 2020 Edition), the stability of the crystalline form A of the compound of formula (I) was investigated under long-term conditions (25 °C/relative humidity at 60%, sealed).

1.5 g of the crystalline form A of the compound of formula (I) was weighed into a double-layer low-density polyethylene bag. Each layer of the low-density polyethylene bag was sealed with a binding tie, and the bag was put into an aluminum foil bag. Samples were taken in months 3 and 6 for analysis, and the results were compared with the preliminary results of day 0. This experiment was repeated twice, each with a different batch of the crystalline form A of the compound of formula (I). The experimental results are shown in Table 5 below.

**Table 5. Solid stability test results of crystalline form A of the compound of formula (I) under long-term conditions (25 °C/relative humidity at 60%, sealed)**

| Conditions | Batch | Time point of sampling | Appearance | Content | Total impurities | Crystalline form (XRPD) |
|---|---|---|---|---|---|---|
| - | 1 | Day 0 | Light green powder | 98.9% | <0.05% | Crystalline form A |
| 25 °C/relative humidity at 60%, sealed | | Month 3 | Light green powder | 99.8% | <0.05% | Crystalline form A |
| | | Month 6 | Light green powder | 99.2% | <0.05% | Crystalline form A |
| - | 2 | Day 0 | White powder | 100.2% | <0.05% | Crystalline form A |
| 25 °C/relative humidity at 60%, sealed | | Month 3 | White powder | 100.4% | <0.05% | Crystalline form A |
| | | Month 6 | White powder | 100.4% | <0.05% | Crystalline form A |

| | | | | | | |
|---|---|---|---|---|---|---|
| Conclusion: The crystalline form A of the compound of formula (I) has good stability under long-term conditions at 25 °C with relative humidity at 60%. | | | | | | |

### Test Example 1: In Vitro Assay for IKZF3 Protein Level in Multiple Myeloma Cells

### Objective:

By using WB method, the modulation of the IKZF3 protein level in multiple myeloma cells MM.1S by the compound of formula (I) at different concentrations was investigated.

### Experimental procedure:

1) MM.1S cells were thawed and passaged twice;
2) The MM.1S cells were seeded into a 6-well plate at 1 × 10⁶ cells per well and then treated with the compound of formula (I) at a certain concentration;
3) After 16 h of treatment, the cultured cell sample was dissolved in RIPA buffer (Sigma-Aldrich) or NETN buffer (150 mM NaCl, 1% NP-40, 50 mM Tris-HCl, pH 8.0) containing a complete histone inhibitor (Roche) placed on ice, and left to stand for 20 min;
4) After centrifugation (rotation speed: 17950 rpm) for 15 min, the supernatant was collected and quantitative determination of protein (Pierce BCA protein assay kit, Thermo) was performed;
5) An equal amount of 20 µg of protein from each sample was separated by SDS-PAGE and transferred to PVDF or nylon membrane (Invitrogen);
6) 5% skim milk powder was added, followed by overnight incubation with primary antibody (anti-IKZF3 (NBP2-24495, Novus Biologicals) and anti-Actin (1844-1, Epitomics)) in 5% BSA at 4 °C; and
7) After a final reaction with HRP-linked secondary antibody (Goat-anti-rabbit IgG (sc-2004, Santa Cruz)) for 1 h, the bands on the membrane were detected with a chemiluminescent substrate (Thermo Scientific).

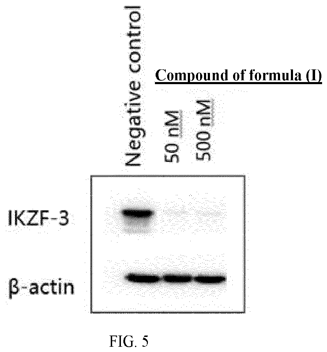

The experimental results are shown in FIG. 5.

### Test Example 2: Evaluation of Antiproliferative Effect in Multiple Myeloma Cell Lines MM.1S and NCI-H929

### Objective:

In this experiment, the inhibition of cell proliferation in multiple myeloma cell lines MM.1S and NCI-H929 by the compound of formula (I) was detected.

### Materials:

1. Cell line and culture method

| Cell line | Tumor type | Characteristics of growth | Culture method |
|---|---|---|---|
| MM.1S | Multiple myeloma | Semi -adhesion | RPMI-1640+10%FBS |
| NCI-H929 | Myeloma | Suspension | RPMI-1640+0.05mM 2-mercaptoethanol+10%FBS |

2. Culture medium and reagent

| Culture medium and reagent | Manufacturer | Catalog number |
|---|---|---|
| RPMI 1640 | GIBCO | 22400-089 |
| Dulbecco's PBS | Hyclone | SH30256.01 |
| FBS | Hyclone | SY30087.03 |
| Antibiotic-antimycotic | GIBCO | 15240-062 |
| 0.25% Trypsin | GIBCO | 25200072 |
| DMSO | SIGMA | D2650 |
| 2-mercaptoethanol | SIGMA | 60-24-2 |

3. Multi-well plate
Greiner CELLSTAR^{®} 96-well plate, flat-bottomed blackboard (with cover and transparent bottom), # 655090.
4. Reagent and instrument for cell viability experiment
(1) Promega CellTiter-Glo luminescence method cell viability assay kit (Promega-G7573).
(2) 2104 EnVision^{®} plate reader, PerkinElmer.

### Experimental procedure:

### 1. Cell culturing

The tumor cell lines were cultured in an incubator at 37 °C with 5% CO₂ under the culture conditions described above. Periodical passaging was performed, and cells growing at log phase were used for plating.

### 2. Cell plating

(1) Cells were stained with trypan blue and viable cells were counted.
(2) The cell concentration was adjusted to an appropriate level.

| **Cell line** | **Density (per 96-well)** |
|---|---|
| MM.1S | 4000 |
| NCI-H929 | 6000 |

(3) 90 µL of cell suspension was added to each well in the culture plate and cell-free medium was added to the blank control wells according to the table above.
(4) The culture plate was incubated overnight in an incubator at 37 °C with 5% CO₂ and 100% relative humidity.

### 3. Preparation of compound storage plate

Preparation of storage plate of mother solution at a concentration 400 times higher than the initial concentration of the compound of formula (I): the compound of formula (I) was diluted from the highest concentration gradient to the lowest concentration with DMSO. It was prepared freshly prior to use.

### 4. Preparation of working solution at a concentration 10 times higher than the initial concentration of the compound of formula (I) and treatment of cells with the compound

(1) Preparation of working solution at a concentration 10 times higher than the initial concentration of the compound of formula (I): 76 µL of the cell culture was added to the V-bottomed 96-well plate, and 4 µL of the compound was pipetted from a storage plate of mother solution at a concentration 200 times higher than the initial concentration of the compound of formula (I) and added to the cell culture in the 96-well plate. 4 µL of DMSO was added to vehicle control and blank control wells. After the compound of formula (I) or DMSO was added, the mixture was pipetted and mixed well, and 78 uL of the cell culture was added to the V-bottomed 96-well plate, and 2 µL of the compound of formula (I) was pipetted from a storage plate of mother solution at a concentration 400 times higher than the initial concentration of the compound of formula (I) and added to the cell culture in the 96-well plate. 2 µL of DMSO was added to vehicle control and blank control wells. After the compound of formula (I) or DMSO was added, the mixture was pipetted and mixed well.
(2) Dosing: 10 µL of working solution at a concentration 10 times higher than the initial concentration of the compound of formula (I) was added to the cell culture plate. 10 µL of DMSO-cell culture mixture was added to the vehicle control and blank control wells.
(3) The 96-well cell plate was put back to the incubator to incubate MM.1S (3-fold diluted, co-incubated with the drug for 5 days) and NCI-H929 (3-fold diluted, co-incubated with the compound of formula (I) for 5 days).

### 5. Cell viability assay using CellTiter-Glo luminescence method

The following procedure was performed according to the instructions of Promega CellTiter-Glo luminescence cell viability assay kit (Promega-G7573).
(1) The CellTiter-Glo buffer was thawed and left to stand until reaching the room temperature.
(2) The CellTiter-Glo substrate was left to stand until reaching the room temperature.
(3) 10 mL of CellTiter-Glo buffer was added to a bottle of CellTiter-Glo substrate to dissolve the substrate, thus preparing the CellTiter-Glo working solution.
(4) Slow vortex shaking was performed to completely dissolve the substrate.
(5) The cell culture plate was taken out and left to stand for 30 min so as to be equilibrated to room temperature.
(6) 50 µL (equal to half the volume of cell culture in each well) of CellTiter-Glo working solution was added to each well. The cell plate was wrapped with aluminum foil to keep out of light.
(7) The plate was shaken on an orbital shaker for 2 min to induce cell lysis.
(8) The culture plate was placed at room temperature for 10 min to stabilize the luminescence signals.
(9) The luminescence signals were detected on 2104 EnVision plate reader.

### 6. Data analysis

The inhibition rates (IRs) of the detected compound of formula (I) were calculated according to the following formula: IR (%) = (RLU vehicle control - RLU compound) / (RLU vehicle control - RLU blank control) × 100%. Inhibition rates of the compound of formula (I) at different concentrations were calculated in Excel, followed by plotting the inhibition curves and calculating relevant parameters including minimum inhibition rate, maximum inhibition rate and IC₅₀ using GraphPad Prism software.

### Experimental results:

The test results are shown in Table **6.**

**Table 6. Inhibition of cell proliferation in the MM.1S and NCI-H929 cell lines by the compound of the present application**

| Compound | MM.1S IC₅₀ (nM) | NCI-H929 IC₅₀ (nM) |
|---|---|---|
| Compound of formula (I) | 0.4 | 0.5 |

### Conclusion:

The compound of formula (I) had an excellent inhibitory effect on cell proliferation in multiple myeloma cell lines MM.1S and NCI-H929.

### Test Example 3: Pharmacokinetic Evaluation in Mice

### Objective:

The test animals in the study were C57BL male mice, and the LC/MS/MS method was used to quantitatively determine the drug concentration in the plasma of the mice at different time points after intravenous injection or oral administration of the test compound, thus evaluating the pharmacokinetic characteristics of the compound of formula (I) in the mice.

### Materials:

C57Balb/C (C57) mice (male, 20-30 g, 7-10 weeks old, Beijing Vital River or Shanghai SLAC).

### Procedures:

Clear or suspended solution of the compound of formula (I) was injected into C57 mice (fasted overnight) via the tail vein or administered intragastrically to C57 mice (fasted overnight). For intravenous injection, 200 µL of blood was each collected by jugular vein puncture at 0 h (before injection) and 0.0833 h, 0.25 h, 0.5 h, 1 h, 2 h, 4 h, 6 h, 8 h and 24 h (after injection), and then placed in an anticoagulation tube added with EDTA-K2 (Jiangsu KANGJIAN Medical Apparatus Co., Ltd.), and the mixture was thoroughly vortex-mixed at 4 °C and centrifuged at 13000 rpm for 10 min; for intragastric administration, blood was collected by jugular vein puncture at 0 h (before administration) and 0.5 h, 1 h, 2 h, 4 h, 6 h, 8 h and 24 h (after administration), and then placed in an anticoagulation tube added with EDTA-K2 (Jiangsu KANGJIAN Medical Apparatus Co., Ltd.), and the mixture was thoroughly vortex-mixed and centrifuged at 13000 rpm for 10 min. The plasma concentration was measured by LC-MS/MS, and the relevant pharmacokinetic parameters were calculated using WinNonlin^{™} Version 6.3 (Pharsight, Mountain View, CA), a pharmacokinetic software, with a non-compartmental model linear-log trapezoidal method.

### Experimental results:

The test results are shown in Table 7.

**Table 7. Pharmacokinetic parameters of the compound of formula (I) of the present application in mice**

| Pharmacokinetic parameters in mice | Intravenous injection (2 mg/kg) | | | Oral administration (10 mg/kg) | | | |
|---|---|---|---|---|---|---|---|
| | Plasma clearance (mL/min/kg) | Half-life (h) | Area under plasma concentration-time curve (0-inf, µM·h) | Peak concentration (µM) | Time to peak (h) | Area under plasma concentration-time curve (0-inf, µM·h) | Bioavailability F (%) |
| Compound of formula (I) | 23.2 | 0.89 | 5.34 | 10.52 | 0.25 | 12.29 | 46.1 |

### Test Example 4: Pharmacokinetic Evaluation in Rats

### Objective:

The test animals in the study were SD male rats, and the LC/MS/MS method was used to quantitatively determine the drug concentration in the plasma of the rats at different time points after intravenous injection or oral administration of the compound of formula (I), thus evaluating the pharmacokinetic characteristics of the compound of formula (I) in the rats.

### Materials:

Sprague Dawley (SD) rats (male, 200-300 g, 7-10 weeks old, Beijing Vital River or Shanghai SLAC).

### Procedures:

Clear solution of the compound of formula (I) was injected into SD rats (fasted overnight) via the tail vein or administered intragastrically to SD rats (fasted overnight). For intravenous injection, 200 µL of blood was each collected by jugular vein puncture at 0 h (before injection) and 0.0833 h, 0.25 h, 0.5 h, 1 h, 2 h, 4 h, 6 h, 8 h and 24 h (after injection), and then placed in an anticoagulation tube added with EDTA-K2 (Jiangsu KANGJIAN Medical Apparatus Co., Ltd.), and the mixture was thoroughly vortex-mixed at 4 °C and centrifuged at 13000 rpm for 10 min; for intragastric administration, blood was collected by jugular vein puncture at 0 h (before administration) and 0.5 h, 1 h, 2 h, 4 h, 6 h, 8 h and 24 h (after administration), and then placed in an anticoagulation tube added with EDTA-K2 (Jiangsu KANGJIAN Medical Apparatus Co., Ltd.), and the mixture was thoroughly vortex-mixed and centrifuged at 13000 rpm for 10 min. The plasma concentration was measured by LC-MS/MS, and the relevant pharmacokinetic parameters were calculated using WinNonlin^{™} Version 6.3 (Pharsight, Mountain View, CA), a pharmacokinetic software, with a non-compartmental model linear-log trapezoidal method.

### Experimental results:

The test results are shown in Table **8.**

**Table 8. Pharmacokinetic parameters of the compound of formula (I) of the present application in rats**

| Pharmacokinetic parameters in rats | Intravenous injection (2 mg/kg) | | | Intragastric administration (10 mg/kg) | | | |
|---|---|---|---|---|---|---|---|
| | Plasma clearance (mL/min/kg) | Half-life (h) | Area under plasma concentration-time curve (0-inf, µM·h) | Peak concentration (µM) | Time to peak (h) | Area under plasma concentration-time curve (0-inf, µM·h) | Bioavailability F (%) |
| Compound of formula (I) | 15.9 | 0.90 | 7.92 | 12.75 | 0.50 | 26.34 | 66.5 |

### Test Example 5: In Vivo Pharmacodynamic Study of the Compound of Formula (I) in Subcutaneous Xenograft Tumor CB-17SCID Model of Human Myeloma MM.1S Cells

Cell culturing: human multiple myeloma cells MM.1S (ATCC^{®} CRL-2974^{™}) were cultured in vitro, in a semi-suspension manner, with an ATCC-formulated RPMI-1640 medium containing 10% fetal calf serum, 100 U/mL penicillin and 100 µg/mL streptomycin in an incubator at 37 °C with 5% CO2. Passages were performed twice a week. At a required number, the cells were taken, counted and inoculated.

Animals: CB-17 SCID mice, female, 6-8 weeks old, weighing 18-20 g.

### Experimental procedure:

0.2 mL (5 × 10⁶ cells) of MM.1S cells (along with matrigel in a volume ratio of 1:1) was subcutaneously inoculated on the right back of each mouse, and the mice were divided into groups for administration after the mean tumor volume was approximately 130 mm³. Seven days constitute an administration cycle, twice daily with a 12 h interval, and the compound of formula (I) was orally administered for a total of four cycles. The compound of formula (I) was administered at a dose of 5 mg/kg, tumor volume was measured twice weekly with a two-dimensional caliper, and the volume was measured in cubic millimeters and calculated according to the following formula: V = 0.5 a × b², where a and b are the long and short diameters, respectively, of the tumor. Anti-tumor efficacy was determined by dividing the mean tumor increase volume of animals treated with the compound of formula (I) by the mean tumor increase volume of untreated animals.

### Experimental results:

The test results are shown in Table **9.**

**Table 9. Test results of the compound of formula (I) in subcutaneous xenograft tumor CB-17 SCID model of human myeloma MM. 1S cells**

| Group | Dosage | Tumor volume (mm³) (Day 0) | Tumor volume (mm³) (Day 24) | TGI (%) (Day 24) |
|---|---|---|---|---|
| Vehicle control | 0 mg/kg | 130±9 | 2,637±332 | / |
| Compound of formula (I) | 5 mg/kg | 131±12 | 14±5 | 105 |

| | | | | |
|---|---|---|---|---|
| TGI: tumor growth inhibition. TGI(%) = [1 - (mean tumor volume of a treatment group at the end of administration - mean tumor volume of the treatment group during administration) / (mean tumor volume of a solvent control group at the end of treatment - mean tumor volume of the solvent control group at the beginning of treatment)] × 100%. | | | | |

## Claims

1. A crystalline form of a compound of formula (I)

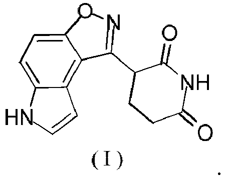

2. The crystalline form of the compound of formula (I) according to claim 1, having diffraction peaks at the following 2θ angles in an X-ray powder diffraction pattern using Cu-Ka radiation: 10.885±0.200°, 16.433±0.200° and 24.417±0.200°.

3. The crystalline form of the compound of formula (I) according to claim 2, having diffraction peaks at the following 2θ angles in an X-ray powder diffraction pattern using Cu-Ka radiation: 10.885±0.200°, 16.433±0.200°, 18.052±0.200°, 18.942±0.200°, 21.124±0.200°, 21.740±0.200°, 24.417±0.200° and 29.034±0.200°.

4. The crystalline form of the compound of formula (I) according to claim 3, having diffraction peaks at the following 2θ angles in an X-ray powder diffraction pattern using Cu-Ka radiation: 10.885±0.200°, 15.303±0.200°, 16.433±0.200°, 18.052±0.200°, 18.942±0.200°, 21.124±0.200°, 21.740±0.200°, 24.417±0.200°, 25.766±0.200°, 26.189±0.200°, 28.741±0.200° and 29.034±0.200°.

5. The crystalline form of the compound of formula (I) according to claim 4, having diffraction peaks at the following 2θ angles in an X-ray powder diffraction pattern using Cu-Ka radiation: 10.885±0.200°, 15.303±0.200°, 16.433±0.200°, 18.052±0.200°, 18.942±0.200°, 21.124±0.200°, 21.740±0.200°, 24.417±0.200°, 25.766±0.200°, 26.189±0.200°, 28.741±0.200°, 29.034±0.200°, 31.241±0.200°, 32.588±0.200°, 32.958±0.200°, 33.123±0.200°, 34.985±0.200°, 36.474±0.200° and 37.811±0.200°.

6. The crystalline form of the compound of formula (I) according to claim 1, wherein the X-ray powder diffraction pattern using Cu-Ka radiation is shown in FIG. 1.

7. The crystalline form of the compound of formula (I) according to claim 1, having a differential scanning calorimetry curve showing an endothermic peak at 260.2 °C.

8. The crystalline form of the compound of formula (I) according to claim 1, having a hygroscopic weight gain of less than 0.2% at 25 °C with relative humidity at 80%.

9. A method for preparing the crystalline form of the compound of formula (I) according to any one of claims 1-8, comprising a step of precipitating the compound of formula (I) from an organic solvent.

10. A crystalline composition, wherein the crystalline form of the compound of formula (I) according to claim 1 makes up 50% or more, preferably 75% or more, more preferably 90% or more, and most preferably 95% or more, of the crystalline composition by weight.

11. A pharmaceutical composition, comprising the crystalline form of the compound of formula (I) according to any one of claims 1-9 or the crystalline composition according to claim 10, and comprising at least one pharmaceutically acceptable carrier.

12. The pharmaceutical composition according to claim 11, selected from the group consisting of pharmaceutical compositions for oral, subcutaneous, intramuscular and intravenous administration.

13. The pharmaceutical composition according to claim 11 or 12, selected from the group consisting of tablets, capsules and granules, or from the group consisting of aqueous and non-aqueous solutions for injection.

14. Use of the crystalline form of the compound of formula (I) according to any one of claims 1-9, the crystalline composition according to claim 10, or the pharmaceutical composition according to any one of claims 11-13 for preparing a medicament for treating a CRBN protein-associated disease, wherein preferably, the CRBN protein-associated disease is selected from multiple myeloma.

15. The crystalline form of the compound of formula (I) according to any one of claims 1-9, the crystalline composition according to claim 10, or the pharmaceutical composition according to any one of claims 11-13 for use in treating a CRBN protein-associated disease, wherein preferably, the CRBN protein-associated disease is selected from multiple myeloma.
